(19) Europäisches Patentamt
European Patent Office
Office européen des brevets

(11) **EP 2 750 730 B1**

(12) **EUROPÄISCHE PATENTSCHRIFT**

(45) Veröffentlichungstag und Bekanntmachung des Hinweises auf die Patenterteilung:
**16.05.2018  Patentblatt 2018/20**

(21) Anmeldenummer: **12755988.8**

(22) Anmeldetag: **31.08.2012**

(51) Int Cl.:
*A61M 1/16* (2006.01)     *A61M 1/36* (2006.01)

(86) Internationale Anmeldenummer:
**PCT/EP2012/066978**

(87) Internationale Veröffentlichungsnummer:
**WO 2013/030350 (07.03.2013 Gazette 2013/10)**

(54) **DIALYSATFLUSS-REGELUNG**

DIALYSATE FLOW CONTROL

RÉGULATION DU FLUX DE DIALYSAT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.09.2011  DE 102011053200**
**01.09.2011  US 201161529965 P**

(43) Veröffentlichungstag der Anmeldung:
**09.07.2014  Patentblatt 2014/28**

(73) Patentinhaber: **Fresenius Medical Care Deutschland GmbH**
**61352 Bad Homburg (DE)**

(72) Erfinder:
• **KOPPERSCHMIDT, Pascal**
**97456 Dittelbrunn (DE)**
• **GAGEL, Alfred**
**96123 Litzendorf (DE)**

(74) Vertreter: **Nordmeyer, Philipp Werner**
**df-mp Dörries Frank-Molnia & Pohlman Patentanwälte Rechtsanwälte PartG mbB Theatinerstraße 16**
**80333 München (DE)**

(56) Entgegenhaltungen:
**EP-A1- 0 911 043          WO-A1-98/32476**
**WO-A1-2007/140993    DE-A1-102006 045 437**
**US-A- 5 744 031          US-A1- 2010 168 925**

**Beschreibung**

Technisches Gebiet

Die Erfindung bezieht sich auf ein Verfahren und eine Vorrichtung zur Regelung des Dialysatflusses in einer Dialysevorrichtung.

Stand der Technik

Dialysevorrichtungen sind gemäß der hier verwendeten Begriffsbedeutung insbesondere Vorrichtungen zur extrakorporalen Blutbehandlung, welche beispielsweise zur Hämodialyse und/ oder Ultrafiltration eingerichtet sein können.

Bei einer Hämodialyse wird in einer Dialysevorrichtung das Blut eines Patienten in einem extrakorporalen Kreislauf durch eine Blutkammer eines Dialysators geleitet. Die Blutkammer ist durch eine semipermeable Membran von einer Dialysatkammer des Dialysators getrennt. Die Dialysatkammer wird von einem Dialysat durchströmt. Das Dialysat enthält Blutelektrolyte in einer Konzentration, die der Konzentration im Blut eines Gesunden entspricht. Während der Behandlung werden das Blut und das Dialysat an den gegenüberliegenden Seiten der semipermeablen Membran im Allgemeinen im Gegenstrom mit einer vorgegebenen Flussrate vorbeigeführt. Die harnpflichtigen Stoffe diffundieren durch die Membran von der Blutkammer in die Dialysatkammer, während gleichzeitig im Blut und im Dialysat vorhandene Elektrolyte von der Kammer höherer Konzentration zur Kammer niedrigerer Konzentration diffundieren.

Zusätzlich kann das Blut entwässert werden, indem an der semipermeablen Membran des Dialysators ein Druckgradient aufgebaut wird, in Folge dessen Wasser aus dem Blut auf die Seite der Dialysatkammer gedrückt wird. Dieser Vorgang wird Ultrafiltration genannt.

Die Wirksamkeit des Dialysators in der Dialysevorrichtung und somit der Blutbehandlung hängt unter anderem auch von der Größe des Dialysatflusses, d.h. der Flussrate des Dialysats, ab. Ein hoher Dialysatfluss kann die Wirksamkeit der Blutbehandlung bis zu einer Obergrenze erhöhen, führt jedoch auch zu einer Erhöhung des Verbrauchs von Dialysat und Energie. Aus der DE 10 2006 045437 A1 sind eine Vorrichtung und ein Verfahren bekannt, wobei die Dialysierflüssigkeitsrate anhand von einem theoretischen Modell und mathematischen Methoden berechnet wird, um die Dialysierflüssigkeitsrate in Bezug auf die zu erreichende Reinigungsleistung über den Dialysator und den Dialysierflüssigkeitsverbrauch ökonomisch günstig vorzugeben.

Es ist folglich wünschenswert und daher eine Aufgabe der Erfindung, die Höhe des Dialysatflusses in einer Dialysevorrichtung derart festlegen zu können, dass die Wirksamkeit der Blutbehandlung möglichst hoch ist und gleichzeitig der Verbrauch von Dialysat und Energie nicht über die Maßen gesteigert wird. Dabei ist es weiterhin wünschenswert, dass eine solche Festlegung der Höhe des Dialysatflusses unabhängig von der Kenntnis des verwendeten Dialysatortyps, des Blutflusses oder bestimmter Patientenparameter, wie beispielsweise dem Vorliegen einer Rezirkulation, vorgenommen werden kann.

Zusammenfassung der Erfindung

Diese Aufgabe wird durch das Verfahren und die Vorrichtung gemäß den unabhängigen Patentansprüchen gelöst. Vorteilhafte Weiterbildungen des Verfahrens und der Vorrichtung sind den jeweiligen abhängigen Patentansprüchen zu entnehmen.

In einem erfindungsgemäßen Verfahren zur Regelung des Dialysatflusses wird eine Blutkammer eines Dialysators mit Blut und eine von der Blutkammer durch eine semipermeable Membran getrennte Dialysatkammer des Dialysators mit einem Dialysat durchströmt. Zur Regelung des Dialysatflusses wird eine durch eine Variation einer Eigenschaft des Dialysats oder des Blutes oder durch eine Änderung des Dialysatflusses bewirkte Änderung des Werts einer Kontrollgröße, die ein Maß für den Stoffaustausch über den Dialysator und somit für die Wirksamkeit des Dialysators ist, bestimmt. Wenn die Änderung des Werts der Kontrollgröße einen Grenzbereich überschreitet, wird der Dialysatfluss erhöht. Hingegen wird der Dialysatfluss reduziert, wenn die Änderung des Werts der Kontrollgröße den Grenzbereich unterschreitet.

Dialysator mit einer Blutkammer zum Durchströmen mit Blut und einer von der Blutkammer durch eine semipermeable Membran getrennten Dialysatkammer zum Durchströmen mit einem Dialysat, Mittel zum Erzeugen des Dialysatflusses durch die Dialysatkammer, eine Einheit, welche zur Bestimmung einer Änderung einer Kontrollgröße eingerichtet ist, wobei die Kontrollgröße ein Maß für den Stoffaustausch über den Dialysator ist und wobei die Bestimmung der Änderung der Kontrollgröße auf einer Modulation der Werte einer Eigenschaft des Dialysats, welche vor und nach dem Dialysator bestimmt werden, beruht, eine Regeleinrichtung zur Regelung des Dialysatflusses durch die Dialysatkammer des Dialysators, und Mittel zur kontinuierlichen Modulation einer Eigenschaft des Dialysats vor dem Eintritt in den Dialysator, wobei die Regeleinrichtung dazu konfiguriert ist, den Dialysatfluss durch die Dialysatkammer zu regeln, wenn eine durch eine kontinuierliche Modulation einer Eigenschaft des Dialysats bewirkte Änderung des Werts der Kontrollgröße nicht innerhalb eines Grenzbereichs liegt, wobei die Regeleinrichtung die Mittel zum Erzeugen des Dialysatflusses anweist, den Dialysatfluss zu erhöhen, wenn die Änderung des Werts der Kontrollgröße den Grenzbereich überschreitet, und den Dialysatfluss zu reduzieren, wenn die Änderung des Werts der Kontrollgröße den Grenzbereich unterschreitet. Wenn die Änderung des Werts der Kontrollgröße durch eine Änderung des Dialysatflusses bewirkt ist, weist die Kontrollgröße vorzugsweise ein Verhältnis oder eine Differenz aus nach und vor der Änderung des Dialysatflusses zuordenbaren Werten einer Eigenschaft des Dialysats oder des Blutes nach dem Dialysator auf. D.h., dass zur Bestimmung des Verhältnisses bzw. der Differenz der Wert einer Eigenschaft des Dialysats oder des Blutes am Austritt aus dem Dialysator von Dialysat bzw. Blut, bei dessen Eintritt in den Dialysator die Änderung des Dialysatflusses bereits stattgefunden hatte, und der Wert von Dialysat bzw. Blut, bei dessen Eintritt in den Dialysator die Änderung des Dialysatflusses noch nicht stattgefunden hatte, verwendet wird.

Wenn die Änderung des Werts der Kontrollgröße durch eine Variation einer Eigenschaft des Dialysats oder des Blutes bewirkt ist, weist die Kontrollgröße vorzugsweise ein Verhältnis oder eine Differenz aus Werten der Eigenschaft nach und vor dem Dialysator auf. D.h., dass bei einer Variation einer Eigenschaft des Dialysats das Verhältnis bzw. die Differenz aus zum einen dem Wert der Eigenschaft des Dialysats nach dem Dialysator oder dem Wert der Eigenschaft des Blutes nach dem Dialysator und zum anderen dem Wert der Eigenschaft des Dialysats vor dem Dialysator oder dem Wert der Eigenschaft des Blutes vor dem Dialysator bestimmt werden kann. Entsprechend gilt bei einer Variation einer Eigenschaft des Blutes, dass das Verhältnis bzw. die Differenz ebenfalls aus zum einen dem Wert der Eigenschaft des Dialysats nach dem Dialysator oder dem Wert der Eigenschaft des Blutes nach dem Dialysator und zum anderen dem Wert der Eigenschaft des Dialysats vor dem Dialysator oder dem Wert der Eigenschaft des Blutes vor dem Dialysator bestimmt werden kann. Vorzugsweise weist die Kontrollgröße das Verhältnis des Werts einer Eigenschaft des Dialysats nach dem Dialysator zu dem Wert der Eigenschaft des Dialysats vor dem Dialysator auf. Eine in dem Ausdruck der Kontrollgröße enthaltene Differenz wird vorzugsweise aus dem Wert einer Eigenschaft des Dialysats nach dem Dialysator und dem Wert der Eigenschaft des Dialysats vor dem Dialysator gebildet.

Die Eigenschaft des Dialysats ist vorzugsweise die elektrische Leitfähigkeit oder die Temperatur des Dialysats und die Eigenschaft des Blutes ist vorzugsweise die elektrische Leitfähigkeit oder die Temperatur des Blutes.

Für eine Bestimmung der Änderung des Werts der Kontrollgröße wird vorzugsweise die Eigenschaft des Dialysats oder des Blutes zumindest für ein Zeitintervall kontinuierlich variiert. Die Änderung des Werts der Kontrollgröße selbst wird vorzugsweise durch eine Ableitung der Kontrollgröße nach dem Dialysatfluss bestimmt.

Der Grenzbereich entspricht vorzugsweise einem Toleranzbereich um einen Wert von 10, 11, 12, 13, 14, 15, 16, 17, 18, 19 oder 20 Prozent des Werts der Kontrollgröße pro Änderung des Dialysatflusses um 100ml/min. Dabei beträgt der Toleranzbereich vorzugsweise ein Prozent des Werts der Kontrollgröße pro Änderung des Dialysatflusses um 100ml/min, so dass bei einem Wert von 10 Prozent des Werts der Kontrollgröße pro Änderung des Dialysatflusses um 100ml/min der Grenzbereich von 9,5 bis 10,5 Prozent des Werts der Kontrollgröße pro Änderung des Dialysatflusses um 100ml/min reicht. Alternativ kann der Toleranzbereich auch null sein, so dass der Grenzbereich auf einen Grenzwert zusammenfällt wie beispielsweise 10, 11, 12, 13, 14, 15, 16, 17, 18, 19 oder 20 Prozent des Werts der Kontrollgröße pro Änderung des Dialysatflusses um 100ml/min.

Vorzugsweise wird eine erfindungsgemäße Vorrichtung zur Regelung eines Dialysatflusses in einer Vorrichtung zur extrakorporalen Blutbehandlung wie beispielsweise eine Dialysevorrichtung eingesetzt.

Die Erfindung wird nachstehend unter Heranziehung der Figuren eingehend erläutert.

Kurze Beschreibung der Zeichnungen

Es zeigt:

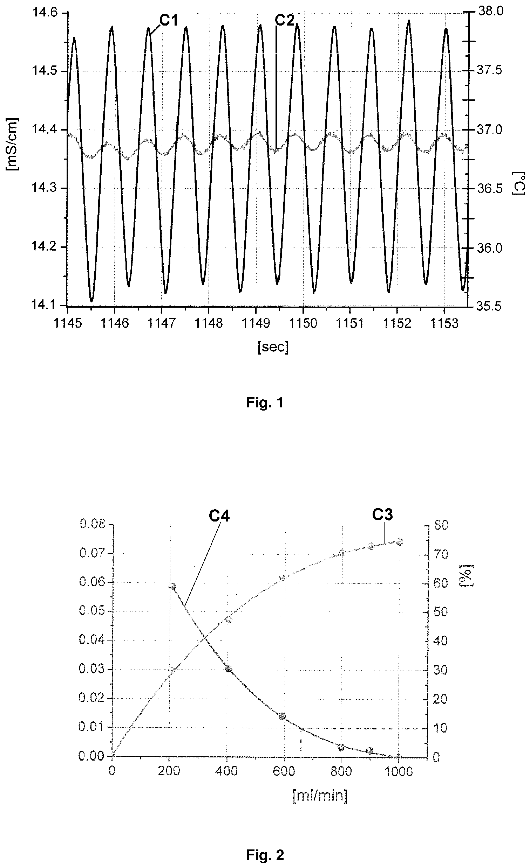

Fig. 1   die zeitlichen Verläufe der elektrischen Leitfähigkeit (linke Ordinatenachse) und der Temperatur (rechte Ordinatenachse) des Dialysats vor (Kurve C1) und nach (Kurve C2) dem Dialysator bei einer Variation der Konzentrateinspritzung bzw. bei einer Variation der Beheizung des Dialysats und einem Dialysatfluss von 800ml/min,

Fig. 2   das Amplitudenverhältnis der Werte der elektrischen Leitfähigkeit bzw. der Temperatur des Dialysats vor und nach dem Dialysator als Funktion des Dialysatflusses (Kurve C3 mit linker Ordinatenachse) sowie die relative Änderung des Amplitudenverhältnisses als Funktion des Dialysatflusses (Kurve C4 mit rechter Ordinatenachse).

Beschreibung der Ausführungsformen

Als ein Maß für die Wirksamkeit eines Dialysators kann die Kontrollgröße

$$k_d = Q_d * (A_{d,out}(Q_d) - A_{d,out}(0)) / A_{d,in}$$

dienen. Dabei beschreibt $Q_d$ den Dialysatfluss in dem Dialysator, während $A_{d,in}$ und $A_{d,out}$ eine Eigenschaft des Dialysats, wie beispielsweise die elektrische Leitfähigkeit oder die Temperatur des Dialysats, vor bzw. nach dem Dialysator beschreiben. $A_{d,out}(0)$ gibt den Wert der Eigenschaft des Dialysats bei fehlender Reinigungsleistung an, beispielsweise bei fehlendem Blutfluss. Bei steigendem Dialysatfluss nimmt die Kontrollgröße $k_d$ zu, sofern eine Reinigungsleistung besteht. Der Zugewinn der Kontrollgröße $k_d$ nimmt jedoch ständig ab. D.h., dass bei einer weiteren Steigerung der Wirksamkeit des Dialysators durch eine Erhöhung des Dialysatflusses der Verbrauch von Dialysat und Energie schnell ansteigt, so dass ab einer bestimmten Grenze eine weitere Erhöhung des Dialysatflusses zur Steigerung der Wirksamkeit des Dialysators nicht mehr sinnvoll ist.

Die Ableitung der durch den Dialysatfluss $Q_d$ dividierten Kontrollgröße $k_d$ nach dem Dialysatfluss $Q_d$ gibt die Änderung der Kontrollgröße $k_d/Q_d$ und somit den normierten Zugewinn der Wirksamkeit des Dialysators aufgrund einer Änderung des Dialysatflusses $Q_d$ an.

$$\varepsilon = \delta(k_d / Q_d) / \delta Q_d = 1 / A_{d,in} * \delta(A_{d,out}(Q_d)) / \delta Q_d$$

In obiger Gleichung wurde vorausgesetzt, dass die Eigenschaft des Dialysats vor dem Dialysator $A_{d,in}$ nicht vom Dialysatfluss $Q_d$ abhängt. Wenn die Eigenschaft des Dialysats beispielsweise die elektrische Leitfähigkeit des Dialysats ist, kann ein vor dem Dialysator konstanter Wert der elektrischen Leitfähigkeit des Dialysats beispielsweise durch eine entsprechende Anpassung der in das Dialysat eingespritzten Menge an Elektrolyten erhalten werden. Dies ist insbesondere wichtig für den Fall, wenn zur Bestimmung der Änderung des Werts der Kontrollgröße die Eigenschaft des Dialysats kontinuierlich variiert wird, wie unten gezeigt wird.

Weiterhin entfällt in der Gleichung für die Ableitung der Kontrollgröße $k_d/Q_d$ nach dem Dialysatfluss $Q_d$ der Term $A_{d,out}(0)$, was eine Bestimmung der Ableitung vereinfacht.

Während einer Blutbehandlung wird der Dialysatfluss variiert und die Änderung der Kontrollgröße $k_d/Q_d$ bestimmt. Dabei kann die Variation des Dialysatflusses von einer gezielten Änderung des Dialysatflusses herrühren oder von sich während der Blutbehandlung ändernden Parametern. Die Bestimmung der Änderung der Kontrollgröße $k_d/Q_d$ kann im Rahmen einer gezielten Änderung des Dialysatflusses durch jeweils eine Bestimmung der Kontrollgröße $k_d/Q_d$ vor und nach der Änderung des Dialysatflusses erfolgen oder durch eine kontinuierliche Bestimmung der Ableitung der Kontrollgröße $k_d/Q_d$ nach dem Dialysatfluss $Q_d$.

Die zuvor bestimmte Änderung der Kontrollgröße wird dann mit einem Grenzwert oder einem Grenzbereich mit einer Obergrenze und einer Untergrenze verglichen. Unterschreitet die Änderung der Kontrollgröße den Grenzwert oder die Untergrenze des Grenzbereichs, wird der Dialysatfluss reduziert. Hingegen wird der Dialysatfluss erhöht, wenn die Änderung der Kontrollgröße den Grenzwert oder die Obergrenze des Grenzbereichs überschreitet. Für den Grenzwert hat sich ein Wert zwischen 10 Prozent und 20 Prozent des Werts der Kontrollgröße pro Änderung des Dialysatflusses um 100ml/ min als besonders vorteilhaft erwiesen. Für jeden dieser Grenzwerte kann auch ein entsprechender Grenzbereich definiert werden, dessen Ober- und Untergrenze einen Toleranzbereich um den Grenzwert angeben. Dabei beträgt der Toleranzbereich vorzugsweise ein Prozent des Werts der Kontrollgröße pro Änderung des Dialysatflusses um 100ml/min, so dass bei einem Grenzwert von 20 Prozent des Werts der Kontrollgröße pro Änderung des Dialysatflusses um 100ml/ min die Unter- und Obergrenze des Grenzbereichs einem Wert von 19,5 bzw. 20,5 Prozent des Werts der Kontrollgröße pro Änderung des Dialysatflusses um 100ml/min entspricht. Durch diese Regelung wird der Dialysatfluss bezogen auf die Wirksamkeit des Dialysators sowie auf den Verbrauch von Dialysat und Energie optimiert.

Zu einem bestimmten Dialysatfluss stellt sich für eine Eigenschaft des Blutes eines Patienten, wie beispielsweise die elektrische Leitfähigkeit, Temperatur oder die Konzentration einer Markersubstanz wie Na-Ionen, Urea, Harnsäure, Beta-2-Mikroglobulin, ein zugehöriges Verhältnis des Werts der entsprechenden Eigenschaft des Dialysats nach dem Dialysator, d.h. beim Austritt aus dem Dialysator, zu dem Wert der entsprechenden Eigenschaft des Dialysats vor dem Dialysator, d.h. beim Eintritt in den Dialysator, ein. Eine Änderung des Dialysatflusses bewirkt eine Änderung des Wertes der entsprechenden Eigenschaft des Dialysats am Austritt aus dem Dialysator. Somit kann eine Bestimmung der Änderung der Kontrollgröße aufgrund einer gezielten Änderung des Dialysatflusses durch eine Bestimmung der Kontrollgröße vor und nach der gezielten Änderung des Dialysatflusses erhalten werden. Hierzu ist jeweils der Wert der entsprechenden Eigenschaft des Dialysats nach dem Austritt aus dem Dialysator zu bestimmen. Demzufolge muss auch bestimmt werden, wann die gezielte Änderung des Dialysatflusses am Austritt aus dem Dialysator angekommen ist, d.h. es muss die hydraulische Verzögerung des Dialysators für den jeweiligen Dialysatfluss bekannt sein, um eine Änderung der Kontrollgröße einer bestimmten Änderung des Dialysatflusses zuordnen zu können. Im Stand der Technik sind Methoden zur Bestimmung der hydraulischen Verzögerung eines Dialysators angegeben. Die gezielte Änderung des Dialysatflusses kann in einer kurzzeitigen, d.h. ein kurzes Zeitintervall andauernden, periodischen oder pulsartigen Änderung der Flussrate des Dialysats bestehen.

Alternativ zur gezielten Änderung des Dialysatflusses kann für die Bestimmung einer Änderung der Kontrollgröße auch die Eigenschaft des Dialysats vor dem Eintritt in den Dialysator kontinuierlich, beispielsweise periodisch, variiert werden. Eine kontinuierliche Modulation des Werts der Eigenschaft des Dialysats am Eintritt in den Dialysator $A_{d,in}$ führt zu einer Modulation des Werts der Eigenschaft des Dialysats am Austritt aus dem Dialysator $A_{d,out}$. Demnach ist in einem eingeschwungenen Zustand bekannt, wie der Wert der Eigenschaft des Dialysats am Austritt aus dem Dialysator zu einem bestimmten Zeitpunkt sein müsste, so dass eine Änderung des Dialysatflusses schnell einer Änderung des Werts der Eigenschaft des Dialysats am Austritt aus dem Dialysator zugeordnet werden kann. Überdies ist somit die Kenntnis der komplexen, weil von der Dialysator-Dialysance, Modulationsfrequenz und Schlauchgeometrie abhängigen, Übertragungsfunktion nicht erforderlich.

Für den Fall, dass die Eigenschaft des Dialysats die elektrische Leitfähigkeit oder die Temperatur des Dialysats ist, sind in der Abbildung gemäß Fig. 1 für eine Dialysebehandlung im Labor (in vitro) die zeitlichen Verläufe der elektrischen Leitfähigkeit (linke Ordinatenachse) bzw. der Temperatur (rechte Ordinatenachse) des Dialysats vor (Kurve C1) und nach (Kurve C2) dem Dialysator bei einem Dialysatfluss von 800ml/min dargestellt. Die nahezu harmonische Variation der elektrischen Leitfähigkeit bzw. der Temperatur des Dialysats am Eintritt in den Dialysator wurde dabei durch eine volumenabhängige Variation der Konzentrateinspritzung, d.h. der Einspritzung von Elektrolyten in das Dialysat, bzw. durch eine Variation der Beheizung des Dialysats erzeugt. Die Änderungen der Konzentrationen der Elektrolyte oder der Konzentration eines einzigen Elektrolyts in dem Dialysat bzw. die thermische Modulation des Dialysats erfolgt dabei innerhalb der für einen Patienten physiologisch unbedenklichen Grenzen.

Aufgrund der Größe des Dialysatorvolumens kommt es im Dialysator zu einer Vermischung zwischen Volumina unterschiedlicher Konzentrationen, d.h. Volumina mit unterschiedlichen Werten der Eigenschaft des Dialysats. Diese Vermischung führt zu einer Dämpfung der Modulation, d.h. der Variation der elektrischen Leitfähigkeit oder der Temperatur des aus dem Dialysator austretenden Dialysats, so dass die Amplitude der elektrischen Leitfähigkeit bzw. der Temperatur des aus dem Dialysator austretenden Dialysats in Abhängigkeit von der Modulationsperiode und dem Dialysatorvolumen reduziert wird. Weiterhin gelangt ein Teil des modulierten Volumens durch die semipermeable Membran in den extrakorporalen Kreislauf (Blutkreislauf), wodurch die Modulation der elektrischen Leitfähigkeit bzw. der Temperatur des aus dem Dialysator austretenden Dialysats zusätzlich reduziert wird. Die Dämpfung der Modulation ist in der Abbildung gemäß Fig. 1 klar durch einen Vergleich der Kurven C1 und C2 zu erkennen, insbesondere durch einen Vergleich der Amplituden.

Die Werte der elektrischen Leitfähigkeit bzw. der Temperatur des Dialysats vor und nach dem Dialysator lassen sich mittels Messzellen erfassen. Aus den Daten der Messzellen lässt sich die Auswirkung einer Änderung des Dialysatflusses unmittelbar erkennen, so dass eine Regelung und somit Optimierung des Dialysatflusses innerhalb kürzester Zeit durchführbar ist.

In der Abbildung gemäß Fig. 2 sind das Amplitudenverhältnis der Werte der elektrischen Leitfähigkeit bzw. der Temperatur des Dialysats vor und nach dem Dialysator als Funktion des Dialysatflusses (Kurve C3 mit linker Ordinatenachse) sowie die relative Änderung des Amplitudenverhältnisses als Funktion des Dialysatflusses (Kurve C4 mit rechter Ordinatenachse) dargestellt.

Die Kurve C3 zeigt, dass der Zuwachs und somit die Änderung des Amplitudenverhältnisses der Werte der elektrischen Leitfähigkeit bzw. der Temperatur des Dialysats vor und nach dem Dialysator mit steigendem Dialysatfluss immer geringer wird. Entsprechend ist der Kurve C4 zu entnehmen, dass die relative Änderung des Amplitudenverhältnisses mit steigendem Dialysatfluss schnell abnimmt. Bereits ab einem Wert des Dialysatflusses von etwa 650ml/ min beträgt die relative Änderung des Amplitudenverhältnisses und somit die Zunahme der Wirksamkeit des Dialysators nur noch weniger als 10 Prozent.

Alternativ zur Bestimmung einer Änderung der Kontrollgröße durch eine Erfassung des Werts der Eigenschaft des Dialysats am Austritt aus dem Dialysator und des Werts der Eigenschaft des Dialysats vor dem Eintritt in den Dialysator, d.h. durch eine Erfassung einer Änderung der Eigenschaft des Dialysats auf der Dialysatseite, kann auch eine im Dialysator vom Dialysat auf das Blut übertragene Eigenschaft auf der Blutseite gemessen werden, d.h. durch Erfassung der entsprechenden Eigenschaft des Blutes am Austritt aus dem Dialysator und vor dem Eintritt in den Dialysator. Zum Beispiel kann die Übertragung eines Temperaturbolus im Dialysat über den Dialysator auf das Blut im venösen Schlauchsystem gemessen werden. Die Kontrollgröße, die auch die Effektivität der Behandlung als Funktion des Dialysatflusses enthält, lautet in diesem Fall

$$k_b = Q_b * (A_{b,out}(Q_d) - A_{b,out}(0)) / A_{b,in}$$

Darin bezeichnet $Q_b$ den Blutfluss, d.h. die Flussrate des Blutes, und $Q_d$ den Dialysatfluss in dem Dialysator.

$A_{b,in}$ beschreibt eine Eigenschaft des Blutes, wie beispielsweise die elektrische Leitfähigkeit oder die Temperatur des Blutes, vor dem Eintritt in den Dialysator und $A_{b,out}$ die entsprechende Eigenschaft des Blutes am Austritt aus dem Dialysator. $A_{b,out}(0)$ gibt den Wert der Eigenschaft des Blutes am Austritt aus dem Dialysator bei fehlender Reinigungsleistung an.

Auch hier ist es vorteilhaft, die Kontrollgröße $k_b$ nicht direkt, sondern die Ableitung der durch den Blutfluss $Q_b$ dividierten Kontrollgröße $k_b$ nach dem Dialysatfluss $Q_d$, also die Änderung der Kontrollgröße $k_b/Q_b$ aufgrund einer Änderung des Dialysatflusses $Q_d$, zu verwenden.

Die Erfindung erlaubt somit auch eine Regelung eines Dialysatflusses mittels einer Variation einer Eigenschaft des Dialysats und Erfassung einer Änderung der entsprechenden Eigenschaft des Blutes auf der Blutseite.

Auch bei einer Bestimmung der Änderung der Kontrollgröße aufgrund einer gezielten Änderung des Dialysatflusses kann die hierzu erforderliche Erfassung einer Änderung einer Eigenschaft des Dialysats oder des Blutes auf der Dialysatseite oder auf der Blutseite durchgeführt werden.

Zur Bestimmung einer Änderung der Kontrollgröße kann alternativ zur Variation einer Eigenschaft des Dialysats vor dem Eintritt in den Dialysator auch eine Eigenschaft des Blutes vor dem Eintritt in den Dialysator kontinuierlich, beispielsweise periodisch, variiert und die Auswirkung der Behandlung auf diese Eigenschaft des Blutes am Austritt aus dem Dialysator auf der Blutseite oder alternativ auf der Dialysatseite gemessen werden. So kann die Variation einer Eigenschaft des Blutes vor dem Eintritt in den Dialysator in einer kleinen, patientenverträglichen Variation der Temperatur des Blutes bestehen. Ebenfalls möglich ist die Variation des Heparineintrags in das Blut vor dem Eintritt in den Dialysator. Bei einer Hämodialyse wird dem Patientenblut vor dem Eintritt in den Dialysator kontinuierlich Heparin oder ein alternatives Antikoagulationsmedikament (Citrat) beigemischt, um eine Zusetzung (Clotting) des Dialysators zu verhindern. Dies geschieht beispielsweise mit einer Spritzenpumpe, die das entsprechende Medikament enthält. Die Flussrate dieser Pumpe (Heparinpumpe) kann entsprechend moduliert werden. Als weitere Alternative kann auch die Substituatsrate moduliert werden. Bei der hierbei vorzugsweise einzusetzenden Prädilution wird dem Blut eines Patienten steriles Substituat vor dem Eintritt in den Dialysator zugeführt. Das überschüssige Wasser wird dem Blut des Patienten durch Ultrafiltration über den Dialysator wieder entzogen. Die Bestandteile des Substituats im Blut, wie beispielsweise Glucose, Natrium, Kalium, Magnesium, Calcium und Bicarbonat, treten innerhalb des Dialysators von der Blutseite über auf die Dialysatseite und sind dort zumindest mittelbar detektierbar, beispielsweise die Elektrolyte durch Leitfähigkeitsmesszellen.

Die Vorrichtung erlaubt somit auch eine Regelung eines Dialysatflusses mittels einer Variation einer Eigenschaft des Blutes und Erfassung einer Änderung der Eigenschaft des Blutes auf der Blutseite oder der Dialysatseite. Die einfachste Methode zur Bestimmung des Amplitudenverhältnisses der Werte der elektrischen Leitfähigkeit des Dialysats vor ($LF_{d,in}$) und nach dem Dialysator ($LF_{d,out}$) ist es, die Amplituden- oder Spitze-Spitze-Werte der elektrischen Leitfähigkeit des Dialysats am Eintritt in den Dialysator ($LF_{d,in}$) und am Austritt aus dem Dialysator ($LF_{d,out}$) miteinander zu vergleichen. Diese Vorgehensweise hat jedoch den Nachteil, dass Rauschen und/oder Störungen unmittelbar zu Messfehlern führen, was wiederum die Genauigkeit des bestimmten Amplitudenverhältnisses reduziert. So betragen in der Kurve C2 der Abbildung gemäß Fig. 1 die Störungen in dem Wert der elektrischen Leitfähigkeit des Dialysats am Austritt aus dem Dialysator circa 10 Prozent. Um die Genauigkeit zu erhöhen, muss folglich über entsprechend viele Halbperioden $T/2$ gemittelt werden.

Daher ist es vorteilhafter mit einer Mittelwertbildung der Signale zu arbeiten, indem die Absolutbeträge der Flächen unter den Leitfähigkeitskurven verglichen werden:

$$\frac{\int_0^{i \cdot T/2} \left| LF_{d,in}(t) - \overline{LF_{d,in}} \right| \cdot dt}{\int_0^{j \cdot T/2} \left| LF_{d,out}(t) - \overline{LF_{d,out}} \right| \cdot dt}$$

Dabei werden alle Messwerte über die Zeit t berücksichtigt, wodurch der statistische Fehler maximal reduziert wird. Dies erfordert jedoch die Ermittlung der Mittelwerte der elektrischen Leitfähigkeit des Dialysats am Eintritt in den Dialysator und am Austritt aus dem Dialysator aus zurückliegenden Halbwellen, was zum einen die gesamte Mittelungsdauer verlängert und zum anderen zu systematischen Messfehlern, wie zum Beispiel bei Drifts, führen kann. Eine andere Möglichkeit wäre es, zunächst alle Messwerte zu speichern und im Nachhinein die Berechnung durchzuführen. Dies erfordert jedoch einen hohen Speicheraufwand.

Daher ist es vorteilhafter mit den Standardabweichungen der Signale bzw. den Effektivwerten der Wechselanteile zu arbeiten, indem die Varianzen der Kurven der elektrischen Leitfähigkeit des Dialysats verglichen werden:

$$\frac{\int_0^{i\cdot T/2}\left(LF_{d,in}(t)-\overline{LF_{d,in}}\right)^2\cdot dt}{\int_0^{j\cdot T/2}\left(LF_{d,out}(t)-\overline{LF_{d,out}}\right)^2\cdot dt}=\frac{\int_0^{i\cdot T/2}\left(LF_{d,in}(t)\right)^2\cdot dt-\dfrac{1}{i\cdot T/2}\cdot\left(\int_0^{i\cdot T/2}LF_{d,in}(t)\cdot dt\right)^2}{\int_0^{j\cdot T/2}\left(LF_{d,out}(t)\right)^2\cdot dt-\dfrac{1}{j\cdot T/2}\cdot\left(\int_0^{j\cdot T/2}LF_{d,out}(t)\cdot dt\right)^2}$$

Hierbei müssen in der Praxis nur die Werte $LF_{d,in}$ / $LF_{d,out}$ und $LF^2_{d,in}$ / $LF^2_{d,out}$ über vollständige Halbwellen summiert werden.

Die vorstehend angegebenen Methoden zur Bestimmung des Amplitudenverhältnisses der Werte der elektrischen Leitfähigkeit des Dialysats vor und nach dem Dialysator sind in analoger Weise auf Werte anderer Eigenschaften des Dialysats oder des Blutes anwendbar.

Der Dialysatfluss lässt sich mit Mitteln wie beispielsweise Pumpen und/oder Ventilen erzeugen und verändern.

Für die Bestimmung der Kontrollgröße aus den Werten der Eigenschaft des Dialysats oder des Blutes vor und nach dem Dialysator sowie zur Bestimmung der Ableitung der Kontrollgröße nach dem Dialysatfluss können beispielsweise programmierbare Mikroprozessoren verwendet werden. Diese sind auch innerhalb einer Regeleinrichtung für die Regelung des Dialysatflusses und die damit verbundene Erzeugung von Steuerbefehlen für Pumpen und/oder Ventile einsetzbar. Die Regeleinrichtung enthält vorteilhafterweise einen Speicher zur Speicherung der Vorgaben für den Grenzwert bzw. den Grenzbereich und für ein Programm, das das erfindungsgemäße Verfahren ausführen kann. Es kann zusätzlich auch vorgesehen sein, dass die Vorgaben für den Grenzwert bzw. den Grenzbereich beim Start oder während der Ausführung des Programms geändert werden können, so dass anstelle eines voreingestellten Werts von beispielsweise 20 Prozent des Werts der Kontrollgröße pro Änderung des Dialysatflusses um 100ml/min auch ein Wert von zum Beispiel 5 Prozent des Werts der Kontrollgröße pro Änderung des Dialysatflusses um 100ml/min eingestellt werden kann. Ebenso kann vorgesehen sein, dass der Toleranzbereich eines Grenzbereichs, d.h. die Ober- und/oder Untergrenze des Grenzbereichs, beim Start oder während der Ausführung des Programms geändert werden kann.

Zusammenfassend ist festzustellen, dass durch die Erfindung der Dialysatfluss in einer Dialysevorrichtung im Hinblick auf die Wirksamkeit des Dialysators und somit auch der Blutbehandlung unabhängig von der Kenntnis des verwendeten Dialysatortyps, des Blutflusses oder bestimmter Patientenparameter optimiert wird. Eine darüber hinausgehende Erhöhung des Dialysatflusses führt zu keiner signifikanten Steigerung der Wirksamkeit des Dialysators.

**Patentansprüche**

1.  Vorrichtung zur Regelung eines Dialysatflusses, aufweisend
    einen Dialysator mit einer Blutkammer zum Durchströmen mit Blut und einer von der Blutkammer durch eine semipermeable Membran getrennten Dialysatkammer zum Durchströmen mit einem Dialysat,
    Mittel zum Erzeugen des Dialysatflusses durch die Dialysatkammer,
    eine Einheit, welche zur Bestimmung einer Änderung einer Kontrollgröße eingerichtet ist, wobei die Kontrollgröße ein Maß für den Stoffaustausch über den Dialysator ist und wobei die Bestimmung der Änderung der Kontrollgröße auf einer Modulation der Werte einer Eigenschaft des Dialysats, welche vor und nach dem Dialysator bestimmt werden, beruht, eine Regeleinrichtung zur Regelung des Dialysatflusses durch die Dialysatkammer des Dialysators, und
    Mittel zur kontinuierlichen Modulation einer Eigenschaft des Dialysats vor dem Eintritt in den Dialysator, wobei die Regeleinrichtung dazu konfiguriert ist, den Dialysatfluss durch die Dialysatkammer zu regeln, wenn eine durch eine kontinuierliche Modulation einer Eigenschaft des Dialysats bewirkte Änderung des Werts der Kontrollgröße nicht innerhalb eines Grenzbereichs liegt, wobei
    die Regeleinrichtung die Mittel zum Erzeugen des Dialysatflusses anweist, den Dialysatfluss zu erhöhen, wenn die Änderung des Werts der Kontrollgröße den Grenzbereich überschreitet, und den Dialysatfluss zu reduzieren, wenn die Änderung des Werts der Kontrollgröße den Grenzbereich unterschreitet.

2.  Vorrichtung nach Anspruch 1, wobei die Eigenschaft des Dialysats die elektrische Leitfähigkeit ist und die Vorrichtung ferner Mittel zum Bestimmen der Werte der elektrischen Leitfähigkeit des Dialysats und/oder des Blutes nach und vor dem Dialysator aufweist oder wobei die Eigenschaft des Dialysats die Temperatur ist und die Vorrichtung ferner Mittel zum Bestimmen der Werte der Temperatur des Dialysats und/oder des Blutes nach und vor dem Dialysator aufweist.

3.  Vorrichtung nach einem der Ansprüche 1 oder 2, wobei die Einheit zur Bestimmung der Kontrollgröße die Änderung des Werts der Kontrollgröße durch eine Ableitung der Kontrollgröße nach dem Dialysatfluss bestimmt.

**4.** Vorrichtung zur extrakorporalen Blutbehandlung aufweisend eine Vorrichtung nach einem der Ansprüche 1 bis 3.


**Claims**

**1.** A device for controlling a dialysate flow, comprising

a dialyzer with a blood chamber for perfusion with blood and a dialysis chamber separated from the blood chamber by a semi-permeable membrane for perfusion with a dialysate,

means to generate the dialysate flow through the dialysis chamber,

a unit configured for the determination of a change of a control factor, wherein the control factor is a measure for the exchange of substances over the dialyzer, and wherein

the determination of the change of the control factor is based on a modulation of the values of a property of the dialysate, wherein the values are determined before and after the dialyzer,

a control facility for controlling the dialysate flow through the dialysis chamber of the dialyzer and

means for continuously modulating a property of the dialysate before the dialyzer,

wherein the control facility is configured to control the dialysate flow through the dialysis chamber, if a change of the value of the control factor caused by a variation of a property of the dialysate or of the blood or by a change of the dialysate flow does not lie within a limit range, wherein

the control facility instructs the means for generating the dialysate flow to increase the dialysate flow if the change of the value of the control factor exceeds the limit range, and to reduce the dialysate flow, if the change of the value of the control factor falls below the limit range.


**2.** The device according to claim 1, wherein the property of the dialysate is the electrical conductivity and the device further has means for determining the values of the electrical conductivity of the dialysate and/or the blood after and before the dialyzer or wherein the property of the dialysate is the temperature and the device further has means for determining the values of the temperature of the dialysate and/or the blood after and before the dialyzer.


**3.** The device according to any one of claims 1 to 2, wherein the unit for determining the control factor determines the change of the value of the control factor by a derivation of the control factor from the dialysate flow.


**4.** A device for the extra-corporeal blood treatment comprising a device according to any one of claims 1 to 3.


**Revendications**

**1.** Dispositif de régulation d'un flux de dialysat, comportant

- un dialyseur avec une chambre à sang destinée au passage du sang et avec une chambre à dialysat destinée au passage d'un dialysat et séparée de la chambre à sang par une membrane semi-perméable,
- des moyens destinés à produire le flux de dialysat dans la chambre à dialysat,
- une unité qui est conçue pour déterminer une variation d'une grandeur de contrôle,
la grandeur de contrôle étant une mesure pour l'échange de substance par l'intermédiaire du dialyseur et la détermination de la variation de la grandeur de contrôle reposant sur une modulation des valeurs d'une propriété du dialysat qui sont déterminées avant et après le dialyseur,
- un dispositif de régulation destiné à réguler le flux de dialysat dans la chambre de dialysat du dialyseur, et
- des moyens destinés à moduler de façon continue une propriété du dialysat avant l'entrée dans le dialyseur,

dans lequel le dispositif de régulation est configuré de manière à réguler le flux de dialysat dans la chambre à dialysat lorsqu'une variation de la valeur de la grandeur de contrôle, variation due à une modulation continue d'une propriété du dialysat, ne se trouve pas à l'intérieur d'une plage limite
et dans lequel le dispositif de régulation ordonne aux moyens destinés à produire le flux de dialysat d'augmenter le flux de dialysat lorsque la variation de la valeur de la grandeur de contrôle devient supérieure à la plage limite et de réduire le flux de dialysat lorsque la variation de la valeur de la grandeur de contrôle devient inférieure à la plage limite.


**2.** Dispositif selon la revendication 1, dans lequel la propriété du dialysat est la conductivité électrique et le dispositif comporte en outre des moyens destinés à déterminer les valeurs de la conductivité électrique du dialysat et/ou du sang après et avant le dialyseur ou dans lequel la propriété du dialysat est la température et le dispositif comporte en outre des moyens destinés à déterminer les valeurs de la température du dialysat et/ou du sang après et avant le dialyseur.

3. Dispositif selon l'une quelconque des revendications 1 ou 2, dans lequel l'unité destinée à déterminer la grandeur de contrôle détermine la variation de la grandeur de contrôle grâce à une dérivée de la grandeur de contrôle par rapport au flux de dialysat.

4. Dispositif destiné au traitement extracorporel du sang comportant un dispositif selon l'une quelconque des revendications 1 à 3.

**Fig. 1**

**Fig. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102006045437 A1